# EUROPÄISCHE PATENTANMELDUNG

(11) **EP 4 018 839 A1**
(43) Veröffentlichungstag der Anmeldung: **29.06.2022**
(21) Anmeldenummer: 20217103.9
(22) Anmeldetag: 23.12.2020
(51) Int. Cl.: A23L 2/38, G01N 33/14

(54) **ANLAGE ZUR HERSTELLUNG EINER VIELZAHL AN MIT EINEM WÄSSRIGEN LEBENSMITTEL BEFÜLLTEN GEBINDEN UND DEREN VERWENDUNG SOWIE VERFAHREN ZUR HERSTELLUNG VON WÄSSRIGEN LEBENSMITTELN**

(71) Anmelder: Red Bull GmbH, 5330 Fuschl am See (AT)
(72) Erfinder: CONCIN, Roland, 5330 Fuschi am See (AT); ÖCHSNER, Ralf, 5330 Fuschi am See (AT); RINDERER, Christian, 5330 Fuschi am See (AT)
(74) Vertreter: Metten, Karl-Heinz

(57) **Zusammenfassung**

Die vorliegende Erfindung betrifft eine Anlage zur Herstellung einer Vielzahl an mit einem wässrigen Lebensmittel befüllten Gebinden oder einen Bestandteil einer solchen Anlage, umfassend ein Massenspektrometer eingerichtet und ausgelegt zur Detektion von Inhaltsstoffen in wässrigen Lebensmitteln oder in Vorstufen hiervon. Ferner betrifft die Erfindung ein Verfahren zur Herstellung von wässrigen Lebensmitteln unter Einsatz einer erfindungsgemäßen Anlage. Außerdem betrifft die Erfindung die Verwendung eines Massenspektrometers, insbesondere eines qTOF-Massenspektrometers, als Bestandteil einer Anlage zur Herstellung einer Vielzahl an mit einem wässrigen Lebensmittel befüllten Gebinden oder zur Detektion von mindestens einem Inhaltsstoff für bzw. in wässrigen Lebensmitteln oder zur in-line-Überwachung von einem oder mehreren Inhaltsstoffen für wässrige Lebensmittel in einer Anlage zur Herstellung einer Vielzahl an mit einem wässrigen Lebensmittel befüllten Gebinden.

## Beschreibung

Die vorliegende Erfindung betrifft eine Anlage zur Herstellung einer Vielzahl an mit einem wässrigen Lebensmittel befüllten Gebinden wie auch einen Bestandteil einer solchen Anlage. Ferner betrifft die Erfindung ein Verfahren zur Herstellung von wässrigen Lebensmitteln. Schließlich betrifft die Erfindung die Verwendung eines Massenspektrometers als Bestandteil einer Anlage zur Herstellung einer Vielzahl an mit einem wässrigen Lebensmittel befüllten Gebinden sowie zur Detektion bzw. in-line-Überwachung von einem oder mehreren Inhaltsstoffen für wässrige Lebensmittel in einer Anlage zur Herstellung einer Vielzahl an mit einem wässrigen Lebensmittel befüllten Gebinden.

Getränke wie Soft- und Energy-Drinks werden schon seit längerem in Highspeed-Produktionsanlagen abgefüllt und versandfertig gemacht. Beispielsweise lassen sich heutzutage ohne weiteres mehrere tausend Gebinde pro Stunde abfüllen. Bei der Herstellung von Getränken, umfassend z.B. das Zusammenführen und Mischen von Einzelkomponenten, die Prozessierung der Ausgangs- bzw. Zwischenprodukte, die Abfüllung von Endprodukten in geeignete Gebinde wie Flaschen oder Dosen und die Durchführung eines Pasteurisierungsschritt, sind vielfältigste hohe Qualitäts- und Hygienestandards fortwährend einzuhalten. Diese Vorgaben stellen hohe Anforderungen sowohl an das Personal, dessen Aus- und Weiterbildung wie auch an die Kontrolle und Wartung der eingesetzten Apparaturen. Stellt man im Nachhinein eine nicht spezifikationskonforme Charge fest, sind mitunter große Mengen an Produkt aufwendig zu entsorgen. Um die Einhaltung von Qualitätsstandards prüfen und dokumentieren zu können, werden regelmäßig stichprobenartig off-line Labortests durchgeführt. Diese Vorgehensweise liefert zwar sehr exakte Analyseergebnisse, ist jedoch aufwendig und liefert in Bezug auf den Zeitpunkt der Probennahme stark zeitversetzte Resultate. Auch lassen sich diese Analyseergebnisse allenfalls relativ grob bestimmten Produktchargen zuordnen. Im Fall nicht spezifikationskonformer Produkte ist eine große Zahl an befüllten Gebinden zu entsorgen.

Der vorliegenden Erfindung hat daher die Aufgabe zugrunde gelegen, eine Anlage zur Herstellung einer Vielzahl an mit einem wässrigen Lebensmittel befüllten Gebinden zur Verfügung zu stellen, die nicht mehr mit den vorangehend geschilderten Nachteilen behaftet ist und die insbesondere eine schnelle und zuverlässige Überprüfung der Qualität bei der Herstellung von Getränken und/oder eine, insbesondere kontinuierliche, Überwachung von für die Herstellung von Getränken wesentlichen Parametern gewährleistet.

Demgemäß wurde eine Anlage zur Herstellung einer Vielzahl an mit einem wässrigen Lebensmittel, insbesondere Getränk, befüllten Gebinden, insbesondere Getränkegebinden, bzw. ein Bestandteil einer solchen Anlage gefunden, umfassend a) mindestens ein Massenspektrometer, insbesondere enthaltend eine Probenleitung, eingerichtet und ausgelegt zur Detektion von Inhaltsstoffen, insbesondere Getränkeinhaltsstoffen, in wässrigen Lebensmitteln oder in Vorstufen hiervon. Besonders gute und zuverlässige Resultate, insbesondere hinsichtlich quantitativer Analysewerte werden hierbei mit einem qTOF-Massenspektrometer oder mit einem Elektrosprayionisation (ESI)-Massenspektrometer erzielt, wobei ESI-qTOF-Massenspektrometer besonders bevorzugt sind. Unter diesen Elektrosprayionisation (ESI)-Massenspektrometern sind wiederum solche mit positivem Ionisierungs-Modus besonders geeignet.

Mit der erfindungsgemäßen apparativen Einbindung eines Massenspektrometers, insbesondere eines qTOF-Massenspektrometers, in eine Anlage zur Herstellung einer Vielzahl an mit einem wässrigen Lebensmittel befüllten Gebinden gelingt die, insbesondere quantitative, in-line-Überwachung von einem oder mehreren Inhaltsstoffen für wässrige Lebensmittel, und zwar überraschender Weise sogar über einen sehr breiten Konzentrationsbereich. Dieser kann sich dabei ohne weiteres von 0,01 mg/l bis 90,0 g/l für die Inhaltsstoffe von flüssigen Lebensmitteln wie Getränken erstrecken. Demgemäß sind sowohl die Hauptbestandteile eines flüssigen Lebensmittels wie auch Kleinkomponenten detektier- und überwachbar.

Die erfindungsgemäße Anlage, insbesondere als Anlage für die Herstellung von Getränken, umfasst in einer zweckmäßigen Ausgestaltung ferner
b) mindestens eine Mischvorrichtung, ausgelegt und eingerichtet zum Mischen von Inhaltsstoffen, insbesondere Getränkeinhaltsstoffen, unter Erhalt eines, insbesondere carbonisierten, wässrigen Lebensmittels, insbesondere einer Getränkemischung, insbesondere enthaltend
   einen Mischbehälter sowie
   eine erste Zuleitung zu diesem Mischbehälter für ein, insbesondere carbonisiertes, wässriges System und
   eine zweite Zuleitung zu diesem Mischbehälter für mindestens einen ersten Inhaltsstoff, insbesondere Getränkeinhaltsstoff, oder eine erste Inhaltsstoffmischung, insbesondere Getränkeinhaltsstoffmischung, sowie
   gegebenenfalls eine dritte Zuleitung zu diesem Mischbehälter für mindestens einen Inhaltsstoff, insbesondere zweiten Getränkeinhaltsstoff, oder eine zweite Inhaltsstoffmischung, insbesondere Getränkeinhaltsstoffmischung, sowie
   gegebenenfalls eine vierte Zuleitung zu diesem Mischbehälter für mindestens einen dritten Inhaltsstoff, insbesondere Getränkeinhaltsstoff, oder eine dritte Inhaltsstoffmischung, insbesondere Getränkeinhaltsstoffmischung, und
c) eine Abfüllvorrichtung, verbunden oder verbindbar über eine Produktions- bzw. Verbindungsleitung mit der mindestens einen Mischvorrichtung gemäß Komponente b), vorzugsweise ausgelegt und eingerichtet zum Einfüllen des Lebensmittels, insbesondere der wässrigen Getränkemischung, erhalten oder erhältlich mit der Mischvorrichtung gemäß Komponente b), in eine Vielzahl an Gebindegrundkörpern, bevorzugt Getränkegebindegrundkörpern, besonders bevorzugt Getränkedosengrundkörpern, jeweils enthaltend einen Gebindeboden, eine Gebindewandung und eine Einfüllöffnung.

In einer weiteren zweckmäßigen Ausgestaltung ist die erfindungsgemäße Anlage ferner ausgestattet mit
d) mindestens eine Verschlussvorrichtung, ausgelegt und eingerichtet zum, insbesondere dichten, Verschließen des mit der Abfüllvorrichtung gemäß Komponente c) befüllten Gebindegrundkörpers, bevorzugt Getränkegebindegrundkörpers, besonders bevorzugt des befüllten Getränkedosengrundkörpers, mit einem Gebindeverschluss, insbesondere einem Getränkedosendeckel.

Bei einer erfindungsgemäßen Anlage wird ein, insbesondere kontinuierlich, reibungsfreier Betrieb auch dadurch gefördert, dass die Produktionsleitung, insbesondere Verbindungsleitung, zwischen dem Mischbehälter der Mischvorrichtung und der Abfüllvorrichtung mit einer Bypass-Leitung ausgestattet ist.

Ein Gegenstand der vorliegenden Erfindung ist damit auch eine solche Vorrichtung, geeignet als Bestandteil einer Anlage zur Herstellung einer Vielzahl an mit einem wässrigen Lebensmittel befüllten Gebinden, die neben einem Mischbehälter einer Mischvorrichtung, einer Abfüllvorrichtung und einer Produktions- bzw. Verbindungsleitung zwischen dem Mischbehälter und der Abfüllvorrichtung auch eine Bypass-Leitung aufweist, die, insbesondere benachbart zum Mischbehälter, von der Produktionsleitung, insbesondere Verbindungsleitung, abgeht und die förderstromabwärts, insbesondere benachbart zur Abfüllvorrichtung, wieder in diese Produktioins- bzw. Verbindungsleitung mündet. Teil dieser Vorrichtung bzw. dieses Bestandteils der erfindungsgemäßen Anlage kann in einer besonders vorteilhaften Ausführungsform das Massenspektrometer enthaltend die Probenleitung sein, welches eingerichtet und ausgelegt ist zur Detektion von Inhaltsstoffen, insbesondere Getränkeinhaltsstoffen, in wässrigen Lebensmitteln oder in Vorstufen hiervon. Hierbei geht in einer weiteren sehr zweckmäßigen Ausführungsform eine Probenentnahmeleitung von der Produktionsleitung, insbesondere Verbindungsleitung, oder der Bypass-Leitung ab. Enthält die genannte Vorrichtung bzw. der genannte Bestandteil oder die erfindungsgemäße Anlage eine mit einer Bypass-Leitung ausgestattete Produktions- bzw. Verbindungsleitung, geht in einer vorteilhaften Ausführungsform die Probenentnahmeleitung von der Produktions- bzw. Verbindungsleitung ab, und zwar bevorzugt in dem Bereich, der sich zwischen dem Abgang und dem Zugang der Bypass-Leitung befindet. Alternativ oder zusätzlich kann die Probenentnahmeleitung auch von der Bypass-Leitung, der Mischvorrichtung gemäß Komponente a) oder der Abfüllvorrichtung abgehen. Über die Probenentnahmeleitung gelangt das entnommene Probenmaterial letztendlich zu der Probenleitung des Massenspektrometers, insbesondere des Flugzeit (qTOF)-Massenspektrometers.

Für viele Anwendungen erhält man besonders dadurch zuverlässige Analyseergebnisse, dass die Probenentnahmeleitung benachbart zu der Abfüllvorrichtung von der Produktionsleitung, insbesondere Verbindungsleitung, abgeht. Dies lässt sich z.B. dadurch bewerkstelligen, dass die Probenentnahmeleitung insbesondere benachbart zum oder im Übergang von der Produktions- bzw. Verbindungsleitung von der Abfüllvorrichtung abgeht.

Die Probenentnahmeleitung verfügt vorteilhafter Weise für viele Anwendungen einen Durchmesser im Bereich von 0,2 bis 100 mm, bevorzugt im Bereich von 5 bis 40 mm. Alternativ sowie insbesondere zusätzlich kann die Probenleitung einen Durchmesser im Bereich von 40 bis 200 µm, bevorzugt im Bereich von 100 bis 150 µm, aufweisen.

Die erfindungsgemäße Anlage bzw. der erfindungsgemäße Bestandteil hiervon ist bevorzugt auch mit einer ersten Mischeinheit, in die die Probenentnahmeleitung mündet, ausgestattet, welche ausgelegt und eingerichtet ist zur Verdünnung des der Produktionsleitung, insbesondere Verbindungsleitung, entnommenen wässrigen Lebensmittels, insbesondere der wässrigen Getränkemischung.

Hierbei kann in einer Ausführungsform die erste Mischeinheit zusätzlich ausgelegt und eingerichtet sein zur Vermengung des der Produktionsleitung, insbesondere Verbindungsleitung, entnommenen, insbesondere verdünnten, wässrigen Lebensmittels, insbesondere der wässrigen Getränkemischung, mit einem wässrigen System, enthaltend mindestens einen Inhaltstoff, insbesondere Getränkeinhaltsstoff, bevorzugt eine Vielzahl an Inhaltsstoffen, insbesondere Getränkeinhaltsstoffen, und besonders bevorzugt sämtliche Inhaltsstoffe, insbesondere Getränkeinhaltsstoffe, in Isotopen-markierter Form als Matrix-Standard.

Damit die erste Mischeinheit sowohl zur Verdünnung der entnommenen Materialprobe als auch zur Beimengung von Inhaltsstoffen in Isotopen-markierter Form als Matrix-Standard eingesetzt werden kann, hat es sich als zweckmäßig erwiesen, eine fünfte Zuleitung, ausgelegt und eingerichtet für den Eintrag von Wasser oder eines wässrigen Verdünnungssystems; mindestens eine sechste Zuleitung, ausgelegt und eingerichtet für den Eintrag des wässrigen Systems, enthaltend den mindestens einen Inhaltstoff, bevorzugt die Vielzahl an Inhaltsstoffen und besonders bevorzugt sämtliche Inhaltsstoffe in Isotopen-markierter Form als Matrix-Standard, und eine Ableitung oder einen Auslass, ausgelegt und eingerichtet für den Übergang zu der Probenleitung, vorzusehen.

In einer weiteren Ausführungsform, insbesondere wenn die erste Mischeinheit ausschließlich dafür vorgesehen ist, die entnommene Materialprobe zu verdünnen, kann die erfindungsgemäße Anlage bzw. dessen Bestandteil eine zweite Mischeinheit aufweisen, ausgelegt und eingerichtet zur Vermengung des der Produktionsleitung, insbesondere Verbindungsleitung, entnommenen und in der ersten Mischeinheit verdünnten wässrigen Lebensmittels, insbesondere der wässrigen Getränkemischung, mit einem wässrigen System, enthaltend mindestens einen Inhaltstoff, insbesondere Getränkeinhaltsstoff, bevorzugt eine Vielzahl an Inhaltsstoffen, insbesondere Getränkeinhaltsstoffen, und besonders bevorzugt sämtliche Inhaltsstoffe, insbesondere Getränkeinhaltsstoffe, in Isotopen-markierter Form als Matrix-Standard. Bei dieser Ausführungsform verfügt die erste Mischeinheit bevorzugt über eine siebte Zuleitung, ausgelegt und eingerichtet für den Eintrag von Wasser oder eines wässrigen Verdünnungssystems, und eine Transferleitung, ausgelegt und eingerichtet für den förderstromabwärtsseitigen Übergang zu der zweiten Mischeinheit.

Bei der vorangehend geschilderten Ausführungsform ist die zweite Mischeinheit bevorzugt mit einer achten Zuleitung für die Einbringung des wässrigen Systems enthaltend die Isotopen-markierten Inhaltsstoffe ausgestattet.

In bevorzugten Ausführungsformen ist die erfindungsgemäße Anlage bzw. der erfindungsgemäße Bestandteil dieser Anlage ausgestattet mit mindestens eine Vorrichtung zur Bestimmung der Durchflussrate und/oder mindestens einen Drucksensor, insbesondere eine Vorrichtung zur Bestimmung der Durchflussrate, in der Probenentnahmeleitung, insbesondere förderstromaufwärts zur Ventileinheit und/oder mit mindestens eine Vorrichtung zur Bestimmung der Durchflussrate und/oder mindestens einen Drucksensor, insbesondere eine Vorrichtung zur Bestimmung der Durchflussrate, in einer Transferleitung von der ersten zur zweiten Mischeinheit und/oder mit mindestens eine Vorrichtung zur Bestimmung der Durchflussrate und/oder mindestens einen Drucksensor, insbesondere eine Vorrichtung zur Bestimmung der Durchflussrate, in mindestens einer Zuleitung zur ersten Mischeinheit und/oder mit mindestens eine Vorrichtung zur Bestimmung der Durchflussrate und/oder mindestens einen Drucksensor, insbesondere eine Vorrichtung zur Bestimmung der Durchflussrate, in mindestens einer Zuleitung zur zweiten Mischeinheit.

Demgemäß kann in einer Ausgestaltung vorgesehen sein, dass in dieser achten Zuleitung kann zur weitergehenden Vereinheitlichung und Reproduzierbarkeit der massenspektroskopisch ermittelten Ergebnisse die Vorrichtung zur Bestimmung der Durchflussrate und/oder mindestens ein Drucksensor, insbesondere eine Vorrichtung zur Bestimmung der Durchflussrate, angeordnet ist. Über die Bestimmung der Durchflussrate bzw. des Drucks in der Zuleitung kann die Menge an wässrigem System enthaltend die Isotopen-markierten Inhaltsstoffe feineingestellt werden.

In entsprechender Weise können zu diesem Zweck eine Vorrichtung zur Bestimmung der Durchflussrate und/oder ein Drucksensor, insbesondere eine Vorrichtung zur Bestimmung der Durchflussrate, in der Leitung, die die erste und die zweite Mischeinheit verbindet, vorgesehen sein.

Insbesondere zur Erzielung reproduzierbarer und/oder qualitativ hochwertiger Ergebnisse hat es sich als zweckmäßig erwiesen, in der Probenentnahmeleitung eine Ventileinheit anzuordnen, die ausgelegt und eingerichtet ist, um einen konstanten Volumenstrom in der Probenentnahmeleitung herzustellen und/oder zu gewährleisten.

Um belastbare massenspektroskopische Analyseergebnisse zu erhalten, hat es sich auch als vorteilhaft erwiesen, mindestens eine Vorrichtung zur Bestimmung der Durchflussrate und/oder mindestens einen Drucksensor, insbesondere eine Vorrichtung zur Bestimmung der Durchflussrate, in der Probenentnahmeleitung vorzusehen. Diese Vorrichtung bzw. dieser Drucksensor ist bevorzugt förderstromaufwärts zur Ventileinheit angebracht. Über die mit dieser Vorrichtung oder dem Drucksensor erhaltenen Werte kann mit Hilfe der Ventileinheit ein besonders konstanter Fluss des entnommenen Probenmaterials zur ersten Mischeinheit sichergestellt werden. Hierdurch gelangt man zu einem hohen Maß an Reproduzierbarkeit.

Alternativ oder zusätzlich können auch die erste, zweite, dritte, vierte, fünfte, sechste und/oder siebte Zuleitung eine Vorrichtung zur Bestimmung der Durchflussrate und/oder mindestens einen Drucksensor, insbesondere eine Vorrichtung zur Bestimmung der Durchflussrate, aufweisen.

Die der Erfindung zugrunde liegende Aufgabe wird demgemäß auch durch einen Bestandteil einer Anlage zur Herstellung einer Vielzahl an mit einem wässrigen Lebensmittel, insbesondere Getränk, befüllten Gebinden, insbesondere Getränkegebinden, gelöst, umfassend oder bestehend aus einem Massenspektrometer, bevorzugt qTOF-Massenspektrometer und besonders bevorzugt ESI-qTOF-Massenspektrometer, enthaltend eine Probenleitung, eingerichtet und ausgelegt zur Detektion von Inhaltsstoffen, insbesondere Getränkeinhaltsstoffen, in wässrigen Lebensmitteln oder in Vorstufen hiervon, einer Produktionsleitung, insbesondere Verbindungsleitung, sowie gegebenenfalls einer von der Produktions- bzw. Verbindungsleitung abgehenden und in die Produktions- bzw. Verbindungsleitung förderstromabwärts mündenden Bypass-Leitung, einer von der Produktionsleitung, insbesondere Verbindungsleitung, oder der Bypass-Leitung abgehenden Probeentnahmeleitung, einer ersten, mit der Probeentnahmeleitung verbundenen oder verbindbaren Mischeinheit, wobei die Probenleitung unmittelbar oder mittelbar mit der ersten Mischeinheit verbunden oder verbindbar ist. In einer weiteren Ausführungsform dieses erfindungsgemäßen Bestandteils kann ferner förderstromabwärts zur ersten Mischeinheit eine zweite Mischeinheit vorgesehen sein, wobei die Probenleitung unmittelbar oder mittelbar mit dieser zweiten Mischeinheit verbunden oder verbindbar ist.

Die erfindungsgemäße Anlage bzw. der erfindungsgemäße Bestandteil hiervon verfügen in einer sehr zweckmäßigen Ausführungsform ferner über mindestens ein Umlenkventil in der Produktionsleitung, insbesondere Verbindungsleitung, oder der Bypass-Leitung förderstromabwärts von der Probenentnahmeleitung, ausgelegt und eingerichtet, um den Zutritt von wässrigem Lebensmittel, insbesondere Getränkeflüssigkeit, zur Abfüllvorrichtung zu verhindern.

Die erfindungsgemäße Anlage stellt bevorzugt eine Anlage zur High-Speed-Herstellung oder einen Bestandteil hiervon dar.

In einer besonders zweckmäßigen Weiterentwicklung der erfindungsgemäßen Anlage bzw. des erfindungsgemäßen Bestandteils dieser Anlage ist in diese bzw. in diesen ferner mindestens ein Infrarotspektrometer, bevorzugt NIR-Spektrometer, jeweils eingerichtet und ausgelegt zur in-line-Detektion von Inhaltsstoffen in bzw. für wässrige Lebensmittel oder Vorstufen hiervon, integriert. Hierbei können Probenvolumina insbesondere auch an den Stellen entnommen und anschließend analysiert werden, wie vorangehend für die geschilderten massenspektroskopischen Untersuchungen ausgeführt. Bevorzugt werden dabei über eine Probenentnahmeleitung, die von der Verbindungsleitung zwischen der Misch- und der Abfüllvorrichtung abgeht, dem Infrarotspektrometer Probenvolumina, insbesondere intervallweise, zugeführt. Bei dem Infrarotspektrometer, bevorzugt NIR-Spektrometer, handelt es sich bevorzugt um ein Fourier-Transformation (FT)-Infrarotspektrometer bzw. FT-NIR-Spektrometer. Das mindestens eine Infrarotspektrometer, insbesondere FT-NIR-Spektrometer, umfasst dabei insbesondere mindestens einen Lichtleiter und eine Messzelle, insbesondere Transmissionsprozesssonde, eingerichtet und ausgelegt, um mit mindestens einem fluiden, insbesondere flüssigen, Medium, bestehend aus oder enthaltend den mindestens einen Inhaltsstoff, oder mit dem wässrigen Lebensmittel in Kontakt zu treten. In einer praktischen Ausgestaltung geht der mindestens eine Lichtleiter in die Messzelle, insbesondere die Transmissionsprozesssonde, über bzw. knüpft an diese an. Diese Messzelle bzw. Transmissionsprozesssonde, z.B. in stabförmiger Ausgestaltung, kann als Bestandteil des Infrarotspektrometers in den mit dem wässrigen Lebensmittel befüllten Behälter bzw. insbesondere auch in eine Zu-, Ab- oder Transferleitungen hineinragen, um die für die qualitative und/oder quantitative Analyse erforderliche Wechselwirkung der Inhaltsstoffe mit der Infrarotstrahlung herbeizuführen. Das mindestens eine Infrarotspektrometer, insbesondere FT-NIR-Spektrometer, umfasst dabei insbesondere auch eine Datenverarbeitungseinrichtung, eingerichtet und ausgelegt, die infrarotspektroskopisch ermittelten Daten der jeweiligen Inhaltsstoffe, insbesondere Getränkeinhaltsstoffe, der fluiden, insbesondere flüssigen, Medien oder des wässrigen Lebensmittels zu speichern und auszuwerten, insbesondere abzugleichen mit in der Datenverarbeitungseinrichtung hinterlegten Referenzdaten für den oder die jeweiligen Inhaltsstoffe. Hierbei kann die Auswertung sowohl qualitativ als auch quantitativ erfolgen. Das mindestens eine Infrarotspektrometer, insbesondere FT-NIR-Spektrometer, ist dabei insbesondere ausgelegt und eingerichtet, den Gehalt an zwei oder mehr Inhaltstoffen, insbesondere Getränkeinhaltstoffen, simultan zu ermitteln. Insbesondere unter Einsatz der Datenverarbeitungseinrichtung gelingt es dabei auch, den mindestens einen Inhaltsstoff in dem flüssigen Medium oder in dem wässrigen Lebensmittel quantitativ zu erfassen. Von weiterem Vorteil der erfindungsgemäßen Anlage bzw. des erfindungsgemäßen Anlagenbestandteils ist auch, dass unter Einbindung des Infrarotspektrometers, insbesondere FT-NIR-Spektrometers, sich der mindestens eine Inhaltsstoff für wässrige Lebensmittel, insbesondere die Vielzahl an Inhaltsstoffen, zusätzlich auch quasi-kontinuierlich infrarotspektroskopisch detektieren lassen, bevorzugt in wiederkehrenden Messintervallen im Bereich von 5 bis 90 Sekunden und besonders bevorzugt im Bereich von 10 bis 20 Sekunden. Durch die zusätzliche Einbindung mindestens eines Infrarotspektrometers, insbesondere FT-NIR-Spektrometers, in die erfindungsgemäße Anlage bzw. den erfindungsgemäßen Anlagenbestandteil lässt sich die Messgenauigkeit über das gesamte Spektrum relevanter Inhaltsstoffe weiter verbessern und die Qualität der Überwachung, insbesondere auch bei Anlagen zur High-Speed-Herstellung nochmals erhöhen.

Die der Erfindung zugrunde liegende Aufgabe wird ebenfalls gelöst durch ein Verfahren zur, insbesondere kontinuierlichen, Herstellung von wässrigen Lebensmitteln, insbesondere Getränken, umfassend
- die Zurverfügungstellung einer erfindungsgemäßen Anlage, insbesondere High-Speed-Produktionsanlage,
- die Zurverfügungstellung von Gebindegrundkörpern, bevorzugt Getränkegebindegrundkörpern, besonders bevorzugt Getränkedosengrundkörpern, enthaltend einen Gebindeboden, eine Gebindewandung und eine Einfüllöffnung,
- die Zurverfügungstellung eines Gebindeverschlusses, insbesondere eines Getränkedosendeckels,
- die Zurverfügungstellung eines, insbesondere carbonisierten, wässrigen Systems,
- die Zurverfügungstellung von mindestens einem, insbesondere einer Vielzahl an Inhaltsstoffen, insbesondere Getränkeinhaltsstoffen, sowie gegebenenfalls die Zurverfügungstellung von, insbesondere carbonisiertem, Wasser oder mindestens einer, insbesondere carbonisierten, wässrigen Zusammensetzung,
- die Zurverfügungstellung von wässrigen Systemen, enthaltend Inhaltsstoffen, insbesondere Getränkeinhaltsstoffe, in Isotopen-markierter Form als Matrix-Standard,
- das Einfüllen eines, insbesondere carbonisierten, wässrigen Lebensmittels, insbesondere einer wässrigen Getränkemischung, in den Gebindegrundkörper, bevorzugt Getränkegebindegrundkörper und besonders bevorzugt Getränkedosengrundkörper, und
- das Verschließen der mit dem wässrigen Lebensmittel, insbesondere der wässrigen Getränkemischung, befüllten Gebindegrundkörper, bevorzugt Getränkegebindegrundkörper und besonders bevorzugt Getränkedosengrundkörper, mit einem Gebindeverschluss, insbesondere einem Dosendeckel, insbesondere Getränkedosendeckel, mittels der Verschlussvorrichtung.

Bei der wässrigen Zusammensetzung kann es sich z.B. um ein Süßungsmittelkonzentrat oder einen Sirup enthaltend ein oder mehrere Süßungsmittel handeln.

Das erfindungsgemäße Verfahren umfasst dabei bevorzugt auch das, insbesondere intervallweise und besonders bevorzugt quasi-kontinuierliche, Ermitteln des Gehalts an dem mindestens einen Inhaltstoff, insbesondere Getränkeinhaltstoff, bevorzugt der Vielzahl an Getränkeinhaltsstoffen, in der wässrigen Mischung, insbesondere Getränkemischung, insbesondere simultan, mit Hilfe des Massenspektrometers, insbesondere qTOF-Massenspektrometers, bevorzugt in konstanten Zeiteinheiten.

Eine quasi-kontinuierliche Ermittlung mit dem erfindungsgemäßen Verfahren bzw. mit der erfindungsgemäßen Anlage oder dem erfindungsgemäßen Anlagenbestandteil umfasst beispielsweise die kontinuierliche Wiederholung bzw. Durchführung von massenspektroskopischen Messungen innerhalb eines Zeitraums von 60 Minuten, bevorzugt innerhalb von 30 Minuten und besonders bevorzugt innerhalb von 10 Minuten. Mit dem erfindungsgemäßen Verfahren bzw. mit der erfindungsgemäßen Anlage oder dem erfindungsgemäßen Anlagenbestandteil können Messungen sogar in einer Taktung von z.B. nur einer oder wenigen Minuten vorgenommen werden. Auf der Basis dieser intervallweisen quasi-kontinuierlichen Datenermittlung gelingt eine nahezu lückenlose Überwachung des Produktionsprozesses, womit ein sehr hohes Maß an Prozesssicherheit gewährleistet werden kann.

Die entnommenen Probenvolumina der wässrigen Mischung, insbesondere Getränkemischung, werden bei dem erfindungsgemäßen Verfahren in einer vorteilhaften Ausführungsvariante über die Probenentnahmeleitung der ersten Mischeinheit, vorzugsweise in stets der gleichen Menge, intervallweise, vorzugsweise in stets gleichen Zeitabständen, zugeführt, und in der ersten Mischeinheit verdünnt wie auch mit einem wässrigen System, enthaltend mindestens einen Inhaltsstoff, insbesondere Getränkeinhaltsstoff, bevorzugt eine Vielzahl an Inhaltsstoffen, insbesondere Getränkeinhaltsstoffen und besonders bevorzugt sämtliche Inhaltsstoffe, insbesondere sämtliche Getränkeinhaltsstoffe, in Isotopen-markierter Form als Matrix-Standard, versetzt.

Alternativ kann vorgesehen sein, dass das entnommene Probenvolumen in der ersten Mischeinheit mit einem wässrigen System, enthaltend mindestens einen Inhaltsstoff, insbesondere Getränkeinhaltsstoff, bevorzugt eine Vielzahl an Inhaltsstoffen, insbesondere Getränkeinhaltsstoffen und besonders bevorzugt sämtliche Inhaltsstoffe, insbesondere sämtliche Getränkeinhaltsstoffe, in Isotopen-markierter Form als Matrix-Standard, versetzt und dieses wässrige System zugleich auch genutzt wird, um das entnommene Probenvolumen zu verdünnen.

Ferner kann alternativ vorgesehen sein, dass das entnommene Probenvolumen in der ersten Mischeinheit mit Wasser oder einem wässrigen Verdünnungssystem verdünnt und dass dieses verdünnte Probenvolumen mit einem wässrigen System, enthaltend mindestens einen Inhaltsstoff, insbesondere Getränkeinhaltsstoff, bevorzugt eine Vielzahl an Inhaltsstoffen, insbesondere Getränkeinhaltsstoffen, und besonders bevorzugt sämtliche Inhaltsstoffe, insbesondere sämtliche Getränkeinhaltsstoffe, in Isotopen-markierter Form als Matrix-Standard, förderstromabwärts in einer zweiten Mischeinheit versetzt wird.

Das der ersten oder der zweiten Mischeinheit entstammende verdünnte wässrige System, enthaltend den mindestens einen Inhaltsstoff, insbesondere Getränkeinhaltsstoff, bevorzugt eine Vielzahl an Inhaltsstoffen, insbesondere Getränkeinhaltsstoffen, und besonders bevorzugt sämtliche Inhaltsstoffe, insbesondere sämtliche Getränkeinhaltsstoffe, in Isotopen-markierter Form als Matrix-Standard, wird über die Probenleitung dem Massenspektrometer, insbesondere qTOF-Massenspektrometer, zugeführt und vermessen.

Mit dem erfindungsgemäßen Verfahren bzw. mit der erfindungsgemäßen Anlage können wässrige Getränkemischungen mit Hilfe des Massenspektrometers, insbesondere qTOF-Massenspektrometers, z.B. auf ihren Gehalt an Zucker bzw. natürlichen Saccharid-Süßstoffen, z.B. Mono- und/oder Disaccharid-Süßstoffen, insbesondere Saccharose, Glucose und/oder, insbesondere und, Fructose, Zitronensäure, Vitaminen, insbesondere Vitamin B2, B3, B5 und/oder, insbesondere und, B6, Taurin, Arginin, Coffein, artifizielle Süßungsmittel, insbesondere Sucralose, Aspartam und/oder, insbesondere und, Acesulfam K, und/oder Ethyl-Vanilin analysiert werden. Mit dem erfindungsgemäßen Verfahren bzw. mit der erfindungsgemäßen Anlage lassen sich grundsätzlich Vitamine, natürliche und artifizielle Süßstoffe sowie Aromastoffe detektieren und damit auch quasi-kontinuierliche überwachen.

Überraschend wurde gefunden, dass sich dabei mindestens zwei Inhaltsstoffe, insbesondere Getränkeinhaltsstoffe, insbesondere die Vielzahl an Inhaltsstoffen, insbesondere Getränkeinhaltsstoffen, im Wesentlichen simultan, vorzugsweise quantitativ, mit Hilfe des Massenspektrometers, insbesondere qTOF-Massenspektrometers, bestimmen lassen.

Besonders genaue quantitative Analyseergebnisse, insbesondere bei simultaner Bestimmung mehrerer Inhaltsstoffe, insbesondere Getränkeinhaltsstoffe, sind mit Hilfe des qTOF-Massenspektrometers zugänglich, wenn bei der Messung die Ionensuppression, Ionisierung und/oder Fragmentierung zum Einsatz kommt.

Bei dem erfindungsgemäßen Verfahren ist von Vorteil, die Entnahme der Probenvolumina der wässrigen Mischung, insbesondere Getränkemischung, aus der Mischvorrichtung oder aus der Produktionsleitung, insbesondere Verbindungsleitung, oder der Bypass-Leitung sowie die Injektion der Probevolumina in das Massenspektrometer, insbesondere qTOF-Massenspektrometer, unter jeweils im Wesentlichen identischen Bedingungen vorzunehmen. Identische Bedingungen können z.B. insbesondere dadurch herbeigeführt werden, dass man den Volumenstrom in den jeweils betroffenen Leitungen, insbesondere Zuleitungen und/oder der Produktions- bzw. Verbindungsleitung sowie insbesondere auch in der Probenentnahmeleitung konstant hält und dass man insbesondere auch stets identische Probenvolumina einsetzt.

In einer bevorzugten Ausführungsvariante sieht das erfindungsgemäße Verfahren vor, dass
der Zeitpunkt der Entnahme eines Probenvolumens über die Probenentnahmeleitung in einer Datenverarbeitungseinrichtung gespeichert wird,
dass die massenspektroskopisch ermittelten, insbesondere quantitativen, Daten der jeweiligen Inhaltsstoffe, insbesondere Getränkeinhaltsstoffe, der wässrigen Mischung, insbesondere Getränkemischung, dieses Probenvolumens in einer Datenverarbeitungseinrichtung gespeichert werden und
dass die massenspektroskopisch für dieses Probenvolumen ermittelten Daten der jeweiligen Inhaltsstoffe, insbesondere Getränkeinhaltsstoffe, der wässrigen Mischung, insbesondere Getränkemischung, in der Datenverarbeitungseinrichtung dem Entnahmezeitpunkt zugeordnet werden. Auf diese Weise können die massenspektroskopisch ermittelten Daten eines Probenvolumens in Korrelation gesetzt werden mit dem in ein Gebinde abgefülltes wässriges Lebensmittel, insbesondere Getränk.

In einer alternativen Ausführungsform kann vorgesehen sein, dass der Messzeitpunkt der massenspektroskopisch für ein Probenvolumen ermittelten, insbesondere quantitativen, Daten der jeweiligen Inhaltsstoffe, insbesondere Getränkeinhaltsstoffe, der wässrigen Mischung, insbesondere Getränkemischung, ermittelt und in einer Datenverarbeitungseinrichtung gespeichert wird,
dass die massenspektroskopisch ermittelten, insbesondere quantitativen, Daten der jeweiligen Inhaltsstoffe, insbesondere Getränkeinhaltsstoffe, der wässrigen Mischung, insbesondere Getränkemischung, dieses Probenvolumens in einer Datenverarbeitungseinrichtung gespeichert werden, und
dass die massenspektroskopisch für dieses Probenvolumen ermittelten Daten der jeweiligen Inhaltsstoffe, insbesondere Getränkeinhaltsstoffe, der wässrigen Mischung, insbesondere Getränkemischung, in der Datenverarbeitungseinrichtung dem Messzeitpunkt zugeordnet werden.

In einer Weiterbildung der vorangehenden Ausführungsform kann vorgesehen sein, dass die massenspektroskopisch für ein Probenvolumen ermittelten und einem Messzeitpunkt zugeordneten Daten der jeweiligen Inhaltsstoffe einem in der Abfüllvorrichtung mit dem wässrigen Lebensmittel, insbesondere der wässrigen Getränkemischung, befüllten Gebindegrundkörper oder einer Mehrzahl an aufeinander folgend mit dem wässrigen Lebensmittel, insbesondere der wässrigen Getränkemischung, befüllten Gebindegrundkörpern zugeordnet werden. Diese Zuordnung wird insbesondere auch dadurch ermöglicht, dass es sich bei massenspektroskopischen Analyse im Wesentlichen um eine in-line-Messung handelt, bei der in kurzen aufeinanderfolgenden Zeitabständen entnommene Probenvolumina, insbesondere quantitativ, vermessen werden können. Messzeitpunkt und Abfüllzeitpunkt können dabei verlässlich in Korrelation gebracht werden. Auf diese Weise ist es ohne weiteres möglich, jedenfalls einer Mehrzahl an aufeinander folgend mit dem wässrigen Lebensmittel, insbesondere der wässrigen Getränkemischung, befüllten Gebindegrundkörpern die massenspektroskopisch für das darin abgefüllte wässrigen Lebensmittel, insbesondere simultan, ermittelten Daten zuzuordnen. Vielfach hat es sich dabei als zweckmäßig erwiesen, die befüllten Gebindegrundkörper mit einem Code zu versehen, mit dem die zu einem Messzeitpunkt massenspektroskopisch ermittelten Daten der jeweiligen Inhaltsstoffe einem in einem befüllten Gebindegrundkörper oder in einer Mehrzahl an aufeinander folgend befüllten Gebindegrundkörpern vorliegenden wässrigen Lebensmittel, insbesondere einer wässrigen Getränkemischung, zuordenbar sind.

Das erfindungsgemäße Verfahren sieht in einer sehr vorteilhaften Ausführungsform ferner vor, dass die massenspektroskopisch zu einem Messzeitpunkt ermittelten, insbesondere quantitativen, Daten der jeweiligen Inhaltsstoffe, insbesondere Getränkeinhaltsstoffe, der wässrigen Mischung, insbesondere der Getränkemischung, eines Probenvolumens in einer Datenverarbeitungseinrichtung mit Soll-Bereichswerten abgeglichen werden. Auf diese Weise kann ermöglicht werden, dass ein mit dem wässrigen Lebensmittel, insbesondere der wässrigen Getränkemischung, befüllte und mit einem Gebindeverschluss verschlossene und insbesondere pasteurisierte Gebindegrundkörper oder eine Mehrzahl an aufeinander folgend mit dem wässrigen Lebensmittel, insbesondere der wässrigen Getränkemischung, befüllte und jeweils mit einem Gebindeverschluss verschlossene Gebindegrundkörper, freigegeben werden,
wenn die massenspektroskopisch ermittelten, insbesondere quantitativen, Daten der jeweiligen Inhaltsstoffe für das in dem verschlossenen Gebindegrundkörper vorliegende wässrige Lebensmittel, insbesondere die wässrige Getränkemischung, oder
wenn die massenspektroskopisch ermittelten, insbesondere quantitativen, Daten der jeweiligen Inhaltsstoffe für das in der Mehrzahl an aufeinander folgenden verschlossenen Gebindegrundkörpern vorliegende wässrige Lebensmittel, insbesondere die wässrige Getränkemischung, innerhalb ihrer jeweiligen hinterlegten Soll-Bereichswerte liegen.

Mit dem erfindungsgemäßen Verfahren gelingt es, als nicht-spezifikationskonform ermittelte abgefüllte und verschlossene Gebinde auf einen sehr geringen Umfang einzugrenzen. Hierdurch kann der Entsorgungsaufwand erheblich verringert werden.

Das erfindungsgemäße Verfahren kann in einer besonders zweckmäßigen auch vorsehen, dass das Umlenkventil geschlossen bleibt oder wird, sobald und/oder solange die massenspektroskopisch für ein Probenvolumen ermittelten quantitativen Daten für sämtliche Inhaltsstoffe, insbesondere Getränkeinhaltsstoffe, der wässrigen Mischung, insbesondere Getränkemischung, dieses Probenvolumens innerhalb ihrer jeweiligen hinterlegten Soll-Bereichswerte liegen. Mit dem erfindungsgemäßen Verfahren wird eine Echtzeitüberwachung der Inhaltsstoffe ermöglicht, wodurch unmittelbar regelnd in den Produktionsablauf eingegriffen werden kann.

Demgemäß sieht das erfindungsgemäße Verfahren in einer bevorzugten Ausführungsform vor, dass der Zutritt der wässrigen Mischung, insbesondere Getränkemischung, zu der Abfüllvorrichtung mit Hilfe des, insbesondere mittels automatischer Aktivierung des, Umlenkventils in der Produktions- bzw. Verbindungsleitung, verhindert wird, sobald die massenspektroskopisch für ein Probenvolumen ermittelten quantitativen Daten für mindestens einen Inhaltsstoff, insbesondere Getränkeinhaltsstoff, der wässrigen Mischung, insbesondere Getränkemischung, dieses Probenvolumens außerhalb seines hinterlegten Soll-Bereichswerts liegt, so dass die wässrige Mischung, insbesondere Getränkemischung nicht in die Abfüllvorrichtung gelangt.

Mit dem erfindungsgemäßen Verfahren kann der Gehalt an dem mindestens einen Inhaltstoff, insbesondere Getränkeinhaltstoff, bevorzugt der Gehalt von mindestens 5 Getränkeinhaltsstoffen, besonders bevorzugt von mindestens 10 Getränkeinhaltsstoffen, in der wässrigen Mischung, insbesondere Getränkemischung, insbesondere simultan, mit Hilfe des Massenspektrometers, insbesondere qTOF-Massenspektrometers, intervallweise, vorzugsweise quasi-kontinuierlich, ermittelt wird, bevorzugt in konstanten Zeiteinheiten. Hierbei kann bei dem erfindungsgemäßen Verfahren das Massenspektrometer, insbesondere qTOF-Massenspektrometer, den Gehalt an Inhaltstoffen, insbesondere Getränkeinhaltstoffen, über einen sehr breiten Konzentrationsbereich, insbesondere in einem Konzentrationsbereich von 0,01 mg/l bis 90,0 g/l, insbesondere simultan, ermitteln.

Mit der vorliegenden Erfindung wird demgemäß ebenfalls abgestellt auf die Verwendung eines Massenspektrometers, insbesondere eines Massenspektrometers ausgestattet mit den Merkmalen, wie vorangehend für die erfindungsgemäße Anlage oder das erfindungsgemäße Verfahren spezifiziert, als Bestandteil einer Anlage zur Herstellung einer Vielzahl an mit einem wässrigen Lebensmittel, insbesondere Getränk, befüllten Gebinden, insbesondere Getränkegebinden. Ferner wird mit der vorliegenden Erfindung abgestellt auf die Verwendung eines Massenspektrometers ausgestattet mit den Merkmalen, wie vorangehend für die erfindungsgemäße Anlage oder das erfindungsgemäße Verfahren spezifiziert, zur, insbesondere quantitativen und besonders bevorzugt auch quasi-kontinuierlichen, Detektion von einem oder mehreren Inhaltsstoffen für wässrige Lebensmittel, insbesondere Getränke, in einer Anlage zur Herstellung einer Vielzahl an mit einem wässrigen Lebensmittel, insbesondere Getränk, befüllten Gebinden, insbesondere Getränkegebinden, oder zur, insbesondere quantitativen und besonders bevorzugt auch quasi-kontinuierlichen, in-line-Überwachung von einem oder mehreren Inhaltsstoffen für wässrige Lebensmittel, insbesondere Getränke, in einer Anlage zur Herstellung einer Vielzahl an mit einem wässrigen Lebensmittel, insbesondere Getränk, befüllten Gebinden, insbesondere Getränkegebinden.

Mit der erfindungsgemäßen Anlage bzw. dem erfindungsgemäßen Anlagenbestandteil und dem erfindungsgemäßen Verfahren können durch Einbindung des Massenspektrometers, insbesondere qTOF-Massenspektrometers, ein oder mehrere Inhaltsstoffe für wässrige Lebensmittel, insbesondere Getränke, in einer Anlage zur Herstellung einer Vielzahl an mit einem wässrigen Lebensmittel, insbesondere Getränk, befüllten Gebinden, insbesondere Getränkegebinden, insbesondere quantitativ, detektiert werden, bei Einsatz mehrerer Inhaltsstoffe sogar simultan, wodurch auch eine, insbesondere quantitative, in-line-Überwachung von einem oder mehreren Inhaltsstoffen für wässrige Lebensmittel, insbesondere Getränke, in einer Anlage zur Herstellung einer Vielzahl an mit einem wässrigen Lebensmittel, insbesondere Getränk, befüllten Gebinden, insbesondere Getränkegebinden, möglich ist. Überraschend wurde auch gefunden, dass das erfindungsgemäße Verfahren bei Anlagen zur High-Speed-Herstellung einer Vielzahl an mit einem wässrigen Lebensmittel befüllten Gebinden, insbesondere Getränkegebinden, zuverlässig eingesetzt werden kann. Durch die Einbindung des Massenspektrometers, insbesondere qTOF-Massenspektrometers, in die erfindungsgemäße Anlage bzw. den erfindungsgemäßen Anlagenbestandteil als in-line-Messwerkzeug lassen sich die Messgenauigkeit über das gesamte Spektrum relevanter Inhaltsstoffe gegenüber bekannten Systemen, die regelmäßig off-line arbeiten, verbessern und die Qualität der Überwachung erhöhen.

Die in der vorstehenden Beschreibung und in den Ansprüchen offenbarten Merkmale der Erfindung können sowohl einzeln aus auch in jeder beliebigen Kombination für die Verwirklichung der Erfindung in ihren verschiedenen Ausführungsformen wesentlich sein.

## Patentansprüche

1. Anlage zur Herstellung, insbesondere zur High-Speed-Herstellung, einer Vielzahl an mit einem wässrigen Lebensmittel, insbesondere Getränk, befüllten Gebinden, insbesondere Getränkegebinden, oder Bestandteil einer solchen Anlage, umfassend
a) ein Massenspektrometer enthaltend eine Probenleitung, eingerichtet und ausgelegt zur Detektion von Inhaltsstoffen, insbesondere Getränkeinhaltsstoffen, in wässrigen Lebensmitteln oder in Vorstufen hiervon.

2. Anlage oder Bestandteil hiervon nach Anspruch 1, **dadurch gekennzeichnet, dass** das Massenspektrometer ein qTOF-Massenspektrometer ist, insbesondere ausgelegt und eingerichtet ist, den Gehalt an Inhaltstoffen, insbesondere Getränkeinhaltstoffen, in einem Konzentrationsbereich von 0,01 mg/l bis 90,0 g/l, insbesondere simultan, zu ermitteln.

3. Anlage oder Bestandteil hiervon nach Anspruch 1 oder 2, **dadurch gekennzeichnet** das
das Massenspektrometer ein Elektrosprayionisation (ESI)-Massenspektrometer, insbesondere mit positivem Ionisierungs-Modus, darstellt.

4. Anlage oder Bestandteil hiervon nach einem der vorangehenden Ansprüche, ferner umfassend
b) eine Mischvorrichtung, ausgelegt und eingerichtet zum Mischen von Inhaltsstoffen, insbesondere Getränkeinhaltsstoffen, unter Erhalt eines, insbesondere carbonisierten, wässrigen Lebensmittels, insbesondere einer Getränkemischung, und
c) eine Abfüllvorrichtung, verbunden oder verbindbar über eine Verbindungsleitung mit der Mischvorrichtung gemäß Komponente b).

5. Anlage oder Bestandteil hiervon nach einem der vorangehenden Ansprüche, ferner umfassend
eine Bypass-Leitung, die, insbesondere benachbart zur Mischvorrichtung, von einer Produktionsleitung, insbesondere der Verbindungsleitung, ausgeht, und die förderstromabwärts, insbesondere benachbart zur Abfüllvorrichtung, in die Produktionsleitung, insbesondere Verbindungsleitung, mündet.

6. Anlage oder Bestandteil hiervon nach einem der vorangehenden Ansprüche,
**gekennzeichnet durch**
mindestens eine Probenentnahmeleitung, insbesondere von der Mischvorrichtung gemäß Komponente a) und/oder der Produktionsleitung, insbesondere der Verbindungsleitung, und/oder der Abfüllvorrichtung und/oder der Bypass-Leitung, insbesondere der Produktions- bzw. Verbindungsleitung oder der Bypass-Leitung, zu einer Mischeinheit oder zu dem Massenspektrometer, insbesondere dem Flugzeit (qTOF)-Massenspektrometer.

7. Anlage oder Bestandteil hiervon nach Anspruch 6, **dadurch gekennzeichnet, dass** die Probenentnahmeleitung benachbart zu einer Abfüllvorrichtung von der Produktionsleitung, insbesondere Verbindungsleitung, abgeht, insbesondere benachbart zum oder im Übergang von der Produktionsleitung, insbesondere Verbindungsleitung, in die Abfüllvorrichtung.

8. Anlage oder Bestandteil hiervon nach einem der vorangehenden Ansprüche,
**gekennzeichnet durch**
eine in der Probenentnahmeleitung angeordnete Ventileinheit, ausgelegt und eingerichtet, um einen konstanten Volumenstrom in der Probenentnahmeleitung herzustellen und/oder zu gewährleisten.

9. Anlage oder Bestandteil hiervon nach einem der vorangehenden Ansprüche,
**gekennzeichnet durch**
eine erste Mischeinheit, in die die Probenentnahmeleitung mündet, ausgelegt und eingerichtet zur Verdünnung des der Produktionsleitung, insbesondere Verbindungsleitung, entnommenen wässrigen Lebensmittels, insbesondere der wässrigen Getränkemischung, wobei die erste Mischeinheit insbesondere zusätzlich ausgelegt und eingerichtet ist zur Vermengung des der Produktionsleitung, insbesondere Verbindungsleitung, entnommenen, insbesondere verdünnten, wässrigen Lebensmittels, insbesondere der wässrigen Getränkemischung, mit einem wässrigen System, enthaltend mindestens einen Inhaltstoff, insbesondere Getränkeinhaltsstoff, bevorzugt eine Vielzahl an Inhaltsstoffen, insbesondere Getränkeinhaltsstoffen, und besonders bevorzugt sämtliche Inhaltsstoffe, insbesondere Getränkeinhaltsstoffe, in Isotopen-markierter Form als Matrix-Standard.

10. Anlage oder Bestandteil hiervon nach Anspruche 9, ferner umfassend eine zweite Mischeinheit, ausgelegt und eingerichtet zur Vermengung des der Produktionsleitung, insbesondere Verbindungsleitung, entnommenen und in der ersten Mischeinheit verdünnten wässrigen Lebensmittels, insbesondere der wässrigen Getränkemischung, mit einem wässrigen System, enthaltend mindestens einen Inhaltstoff, insbesondere Getränkeinhaltsstoff, bevorzugt eine Vielzahl an Inhaltsstoffen, insbesondere Getränkeinhaltsstoffen, und besonders bevorzugt sämtliche Inhaltsstoffe, insbesondere Getränkeinhaltsstoffe, in Isotopen-markierter Form als Matrix-Standard.

11. Anlage oder Bestandteil hiervon nach einem der vorangehenden Ansprüche,
**gekennzeichnet durch**
mindestens eine Vorrichtung zur Bestimmung der Durchflussrate und/oder mindestens einen Drucksensor, insbesondere eine Vorrichtung zur Bestimmung der Durchflussrate, in der Probenentnahmeleitung, insbesondere förderstromaufwärts zur Ventileinheit und/oder
mindestens eine Vorrichtung zur Bestimmung der Durchflussrate und/oder mindestens einen Drucksensor, insbesondere eine Vorrichtung zur Bestimmung der Durchflussrate, in einer Transferleitung von der ersten zur zweiten Mischeinheit und/oder
mindestens eine Vorrichtung zur Bestimmung der Durchflussrate und/oder mindestens einen Drucksensor, insbesondere eine Vorrichtung zur Bestimmung der Durchflussrate, in mindestens einer Zuleitung zur ersten Mischeinheit und/oder mindestens eine Vorrichtung zur Bestimmung der Durchflussrate und/oder mindestens einen Drucksensor, insbesondere eine Vorrichtung zur Bestimmung der Durchflussrate, in mindestens einer Zuleitung zur zweiten Mischeinheit.

12. Anlage oder Bestandteil hiervon nach einem der vorangehenden Ansprüche, ferner umfassend
mindestens ein Umlenkventil in der Produktionsleitung, insbesondere Verbindungsleitung, oder der Bypass-Leitung förderstromabwärts von der Probenentnahmeleitung, ausgelegt und eingerichtet, um den Zutritt von wässrigem Lebensmittel, insbesondere Getränkeflüssigkeit, zur Abfüllvorrichtung zu verhindern.

13. Bestandteil einer Anlage zur Herstellung einer Vielzahl an mit einem wässrigen Lebensmittel, insbesondere Getränk, befüllten Gebinden, insbesondere Getränkegebinden, insbesondere einer Anlage nach einem der vorangehenden Ansprüche, umfassend oder bestehend aus
einem Massenspektrometer, bevorzugt qTOF-Massenspektrometer und besonders bevorzugt ESI-qTOF-Massenspektrometer, enthaltend eine Probenleitung, eingerichtet und ausgelegt zur Detektion von Inhaltsstoffen, insbesondere Getränkeinhaltsstoffen, in wässrigen Lebensmitteln oder in Vorstufen hiervon, einer Produktionsleitung, insbesondere Verbindungsleitung, sowie gegebenenfalls einer von der Produktions- bzw. Verbindungsleitung abgehenden und in die Produktions- bzw. Verbindungsleitung förderstromabwärts mündenden Bypass-Leitung,
einer von der Produktionsleitung, insbesondere Verbindungsleitung, oder der Bypass-Leitung abgehenden Probeentnahmeleitung,
mindestens einer mit der Probeentnahmeleitung verbundenen oder verbindbaren Mischeinheit,
wobei die Probenleitung unmittelbar oder mittelbar mit der mindestens einen Mischeinheit verbunden oder verbindbar ist.

14. Anlage oder Bestandteil hiervon nach einem der vor vorangehenden Ansprüche, ferner umfassend
mindestens ein Infrarotspektrometer, insbesondere FT-NIR-Spektrometer, eingerichtet und ausgelegt zur in-line-Detektion von Inhaltsstoffen in wässrigen Lebensmitteln oder in Vorstufen hiervon.

15. Verfahren zur, insbesondere kontinuierlichen, Herstellung von wässrigen Lebensmitteln, insbesondere Getränken wie Energy- oder Softdrinks, umfassend
- die Zurverfügungstellung einer Anlage, insbesondere High-Speed-Anlage, gemäß einem oder mehreren der vorangehenden Patentansprüche,
- die Zurverfügungstellung von Gebindegrundkörpern, bevorzugt Getränkegebindegrundkörpern, besonders bevorzugt Getränkedosengrundkörpern, enthaltend einen Gebindeboden, eine Gebindewandung und eine Einfüllöffnung,
- die Zurverfügungstellung eines Gebindeverschlusses, insbesondere eines Getränkedosendeckels,
- die Zurverfügungstellung eines, insbesondere carbonisierten, wässrigen Systems,
- die Zurverfügungstellung von mindestens einem, insbesondere einer Vielzahl an Inhaltsstoffen, insbesondere Getränkeinhaltsstoffen, sowie gegebenenfalls die Zurverfügungstellung von, insbesondere carbonisiertem, Wasser oder mindestens einer insbesondere carbonisierten, wässrigen Zusammensetzung,
- die Zurverfügungstellung von wässrigen Systemen, enthaltend Inhaltsstoffe, insbesondere Getränkeinhaltsstoffe, in Isotopen-markierter Form als Matrix-Standard,
- das Einfüllen eines, insbesondere carbonisierten, wässrigen Lebensmittels, insbesondere einer wässrigen Getränkemischung, in den Gebindegrundkörper, bevorzugt Getränkegebindegrundkörper und besonders bevorzugt Getränkedosengrundkörper, und
- das Verschließen der mit dem wässrigen Lebensmittel, insbesondere der wässrigen Getränkemischung, befüllten Gebindegrundkörper, bevorzugt Getränkegebindegrundkörper und besonders bevorzugt Getränkedosengrundkörper, mit einem Gebindeverschluss, insbesondere einem Dosendeckel, insbesondere Getränkedosendeckel, mittels einer Verschlussvorrichtung.

16. Verfahren nach Anspruch 15, ferner umfassend
das, bevorzugt intervallweise und besonders bevorzugt quasi-kontinuierliche, Ermitteln des Gehalts an dem mindestens einen Inhaltstoff, insbesondere Getränkeinhaltstoff, bevorzugt der Vielzahl an Getränkeinhaltsstoffen, in der wässrigen Mischung, insbesondere Getränkemischung, insbesondere simultan, mit Hilfe des Massenspektrometers, insbesondere qTOF-Massenspektrometers, bevorzugt in konstanten Zeiteinheiten.

17. Verfahren nach Anspruch 15 oder 16, **dadurch gekennzeichnet, dass**
die wässrige Getränkemischung mit Hilfe des Massenspektrometers, insbesondere qTOF-Massenspektrometers, analysiert wird auf ihren Gehalt an Zucker, insbesondere Saccharose, Glucose und/oder Fructose, Zitronensäure, Vitaminen, insbesondere Vitamin B2, B3, B5 und/oder B6, Taurin, Arginin, Coffein, artifizielle Süßungsmittel, insbesondere Sucralose, Aspartam und/oder Acesulfam K, und/oder Ethyl-Vanillin.

18. Verfahren nach einem der Ansprüche 15 bis 17, **dadurch gekennzeichnet, dass** mindestens zwei Inhaltsstoffe, insbesondere Getränkeinhaltsstoffe, insbesondere die Vielzahl an Inhaltsstoffen, insbesondere Getränkeinhaltsstoffen, im Wesentlichen simultan, vorzugsweise quantitativ, mit Hilfe des Massenspektrometers, insbesondere qTOF-Massenspektrometers, bestimmt werden.

19. Verfahren nach einem der Ansprüche 15 bis 18, **dadurch gekennzeichnet, dass**
der Messzeitpunkt der massenspektroskopisch für ein Probenvolumen ermittelten, insbesondere quantitativen, Daten der jeweiligen Inhaltsstoffe, insbesondere Getränkeinhaltsstoffe, der wässrigen Mischung, insbesondere Getränkemischung, ermittelt und in einer Datenverarbeitungseinrichtung gespeichert wird,
dass die massenspektroskopisch ermittelten, insbesondere quantitativen, Daten der jeweiligen Inhaltsstoffe, insbesondere Getränkeinhaltsstoffe, der wässrigen Mischung, insbesondere Getränkemischung, dieses Probenvolumens in einer Datenverarbeitungseinrichtung gespeichert werden,
dass die massenspektroskopisch für dieses Probenvolumen ermittelten Daten der jeweiligen Inhaltsstoffe, insbesondere Getränkeinhaltsstoffe, der wässrigen Mischung, insbesondere Getränkemischung, in der Datenverarbeitungseinrichtung dem Messzeitpunkt zugeordnet werden.

20. Verfahren nach Anspruch 19, **dadurch gekennzeichnet, dass**
die massenspektroskopisch für ein Probenvolumen ermittelten und einem Messzeitpunkt zugeordneten Daten der jeweiligen Inhaltsstoffe einem in der Abfüllvorrichtung mit dem wässrigen Lebensmittel, insbesondere der wässrigen Getränkemischung, befüllten Gebindegrundkörpern oder einer Mehrzahl an aufeinander folgend mit dem wässrigen Lebensmittel, insbesondere der wässrigen Getränkemischung, befüllten Gebindegrundkörpern zugeordnet werden.

21. Verfahren nach einem der Ansprüche 15 bis 20, **dadurch gekennzeichnet, dass**
die massenspektroskopisch zu einem Messzeitpunkt ermittelten, insbesondere quantitativen, Daten der jeweiligen Inhaltsstoffe, insbesondere Getränkeinhaltsstoffe, der wässrigen Mischung, insbesondere der Getränkemischung, eines Probenvolumens in einer Datenverarbeitungseinrichtung mit Soll-Bereichswerten abgeglichen werden.

22. Verfahren nach Anspruch 21, **dadurch gekennzeichnet, dass**
das Umlenkventil geschlossen bleibt oder wird, sobald und/oder solange die massenspektroskopisch für ein Probenvolumen ermittelten quantitativen Daten für sämtliche Inhaltsstoffe, insbesondere Getränkeinhaltsstoffe, der wässrigen Mischung, insbesondere Getränkemischung, dieses Probenvolumens innerhalb ihrer jeweiligen hinterlegten Soll-Bereichswerte liegen, und/oder, insbesondere und,
dass
der Zutritt der wässrigen Mischung, insbesondere Getränkemischung, zu der Abfüllvorrichtung mit Hilfe des, insbesondere mittels automatischer Aktivierung des, Umlenkventils in der Produktionsleitung, insbesondere Verbindungsleitung, verhindert wird, sobald die massenspektroskopisch für ein Probenvolumen ermittelten quantitativen Daten für mindestens einen Inhaltsstoff, insbesondere Getränkeinhaltsstoff, der wässrigen Mischung, insbesondere Getränkemischung, dieses Probenvolumens außerhalb seines hinterlegten Soll-Bereichswerts liegt, so dass die wässrige Mischung, insbesondere Getränkemischung nicht in die Abfüllvorrichtung gelangt.

23. Verfahren nach einem der Ansprüche 15 bis 22, **dadurch gekennzeichnet, dass**
der Gehalt an dem mindestens einen Inhaltsstoff, insbesondere Getränkeinhaltstoff, bevorzugt der Gehalt von mindestens 5 Getränkeinhaltsstoffen, besonders bevorzugt von mindestens 10 Getränkeinhaltsstoffen, in der wässrigen Mischung, insbesondere Getränkemischung, insbesondere simultan, mit Hilfe des Massenspektrometers, insbesondere qTOF-Massenspektrometers, intervallweise, insbesondere quasi-kontinuierlich, ermittelt wird, bevorzugt in konstanten Zeiteinheiten.

24. Verfahren nach einem der Ansprüche 15 bis 23, **dadurch gekennzeichnet, dass**
das Massenspektrometer, insbesondere qTOF-Massenspektrometer, ausgelegt und eingerichtet ist, den Gehalt an Inhaltstoffen, insbesondere Getränkeinhaltstoffen, in einem Konzentrationsbereich von 0,01 mg/l bis 90,0 g/l, insbesondere simultan, zu ermitteln.

25. Verwendung eines Massenspektrometers, insbesondere eines Massenspektrometers ausgestattet mit den in den Ansprüchen 1 bis 14 und/oder 15 bis 23 genannten Merkmalen, als Bestandteil einer Anlage zur Herstellung einer Vielzahl an mit einem wässrigen Lebensmittel, insbesondere Getränk wie Energy- oder Softdrink, befüllten Gebinden, insbesondere Getränkegebinden.

26. Verwendung eines Massenspektrometers, insbesondere eines Massenspektrometers ausgestattet mit den in den Ansprüchen 1 bis 14 und/oder 15 bis 24 genannten Merkmalen, zur, insbesondere quantitativen und/oder quasi-kontinuierlichen, Detektion von einem oder mehreren Inhaltsstoffen für wässrige Lebensmittel, insbesondere Getränke wie Energy- oder Softdrinks, in einer Anlage zur Herstellung einer Vielzahl an mit einem wässrigen Lebensmittel, insbesondere Getränk, befüllten Gebinden, insbesondere Getränkegebinden, oder zur, insbesondere quantitativen und/oder quasi-kontinuierlichen, in-line-Überwachung von einem oder mehreren Inhaltsstoffen für wässrige Lebensmittel, insbesondere Getränke, in einer Anlage zur Herstellung einer Vielzahl an mit einem wässrigen Lebensmittel, insbesondere Getränk, befüllten Gebinden, insbesondere Getränkegebinden.

27. Verwendung nach Anspruch 25 oder 26, **dadurch gekennzeichnet, dass**
das Massenspektrometer ein qTOF-Massenspektrometer, insbesondere ein qToF-Massenspektrometer mit Elektrosprayionisation, ist.

28. Verwendung nach einem der Ansprüche 25 bis 27, **dadurch gekennzeichnet, dass**
die Anlage zur Herstellung einer Vielzahl an mit einem wässrigen Lebensmittel befüllten Gebinden, insbesondere Getränkegebinden, eine Anlage zur High-Speed-Herstellung darstellt.

29. Verwendung nach einem der Ansprüche 25 bis 28, **dadurch gekennzeichnet, dass**
das qTOF-Massenspektrometer ausgelegt und eingerichtet ist, den Gehalt an Inhaltstoffen, insbesondere Getränkeinhaltstoffen, in einem Konzentrationsbereich von 0,01 mg/l bis 90,0 g/l, insbesondere simultan, zu ermitteln.
